# EUROPEAN PATENT SPECIFICATION

(11) **EP 3 838 203 B1**
(45) Date of publication and mention of the grant of the patent: **29.11.2023**
(21) Application number: 20214687.4
(22) Date of filing: 16.12.2020
(51) Int. Cl.: A61B 18/12, A61M 1/00, F04B 17/03, F04B 23/04

(54) **SURGICAL GENERATOR INCORPORATING MOTOR-DRIVEN, MULTI-OUTPUT SURGICAL PUMP ASSEMBLY**
CHIRURGISCHER GENERATOR MIT MOTORGETRIEBENER CHIRURGISCHER PUMPENANORDNUNG MIT MEHRFACHAUSGABE
GÉNÉRATEUR CHIRURGICAL INCORPORANT UN ENSEMBLE MOTORISÉ DE POMPES CHIRURGICALES À SORTIES MULTIPLES

(30) Priority: 21.12.2019 US 201962952245 P; 06.10.2020 US 202017064122
(43) Date of publication of application: 23.06.2021
(73) Proprietor: Covidien LP, Mansfield, MA 02048 (US)
(72) Inventor: HRENCHIR, Paul, Boulder, Colorado 80301 (US); AQUINO, Allan G., Longmont, Colorado 80301 (US)
(74) Representative: Maschio, Antonio

(56) References cited:
- EP-A2- 0 275 214
- WO-A1-2008/030872
- WO-A1-2019/152824
- US-A1- 2004 204 679
- US-A1- 2006 051 217
- US-A1- 2010 116 375
- US-A1- 2019 015 096
- US-B1- 9 316 216

## Description

### FIELD

The present disclosure relates to surgical pump assemblies and, more particularly, to a multi-output, motor-driven surgical pump assembly and surgical generator incorporating the same.

### BACKGROUND

Surgical pumps are utilized for a variety of different purposes during the course of surgical procedures. For example, surgical pumps may be utilized for irrigation, aspiration, smoke evacuation, insufflation, to inflate/deflate an expandable structure, to circulate fluid in an open and/or closed loop, etc. In some surgical procedures, multiple pump activations are utilized simultaneously, consecutively, alternatingly, and/or overlapping. Often times, these multiple pump activations require different pump outputs to provide different operating parameters, e.g., flow rates, pressures, etc.

Surgical generators are utilized to power surgical instruments and/or provide energy to surgical instruments to enable the application of energy, e.g., electrosurgical (monopolar and/or bipolar radiofrequency (RF)) energy, ultrasonic energy, thermal energy, microwave energy, etc., from the surgical instruments to tissue.

A surgical generator according to the preamble of claim 1 is known from US 2006/051217 A1.

### SUMMARY

As used herein, the term "distal" refers to the portion that is described which is further from a user, while the term "proximal" refers to the portion that is being described which is closer to a user. Further, any or all of the aspects described herein, to the extent consistent, may be used in conjunction with any or all of the other aspects described herein.

The invention is defined in claim 1.

Provided in accordance with aspects of the present disclosure is a surgical pump assembly including a motor configured to provide a motor output, a transmission assembly configured to receive the motor output and provide a plurality of transmission outputs, and a plurality of pumps. Each pump of the plurality of pumps is configured to receive one of the transmission outputs of the plurality of transmission outputs.

In an aspect of the present disclosure, at least one of the transmission outputs of the plurality of transmission outputs is variable independent of the other transmission outputs of the plurality of transmission outputs.

In another aspect of the present disclosure, at least two of the transmission outputs of the plurality of transmission outputs are different from one another.

In another aspect of the present disclosure, the at least two different transmission outputs of the plurality of transmission outputs drive corresponding at least two pumps of the plurality of pumps in different manners.

In yet another aspect of the present disclosure, the at least two different transmission outputs of the plurality of transmission outputs are different rotational speeds.

In still another aspect of the present disclosure, the transmission assembly includes a plurality of transmissions. Each transmission of the plurality of transmissions is coupled between the motor and one of the pumps of the plurality of pumps.

In still yet another aspect of the present disclosure, at least two transmissions of the plurality of transmissions define different input to output ratios.

In another aspect of the present disclosure, at least one of the transmissions of the plurality of transmissions is controllable to vary the input to output ratio thereof. In aspects, at least two of the transmissions of the plurality of transmissions are independently controllable to independently vary the input to output ratios thereof.

In still another aspect of the present disclosure, a controller is disposed in communication with the transmission assembly and configured to instruct the transmission assembly to independently vary at least one of the transmission outputs of the plurality of transmission outputs.

Also provided in accordance with aspects of the present disclosure is a surgical generator. The surgical generator include the surgical pump assembly according to any of the aspects detailed above or otherwise herein. The surgical generator further includes at least one energy module configured to generate an energy-delivery signal and at least one energy port configured to connect to a surgical instrument to deliver the energy-delivery signal thereto.

### BRIEF DESCRIPTION OF THE DRAWINGS

The above and other aspects and features of the present disclosure will become more apparent in light of the following detailed description when taken in conjunction with the accompanying drawings wherein like reference numerals identify similar or identical elements.
FIG. 1 is a side view of a surgical system provided in accordance with the present disclosure including a surgical generator and first and second surgical instruments selectively connectable to the surgical generator; and
FIG. 2 is a block diagram of a surgical pump provided in accordance with the present disclosure and configured for use with the surgical generator of FIG. 1, another surgical component, or as a stand-alone device.

### DETAILED DESCRIPTION

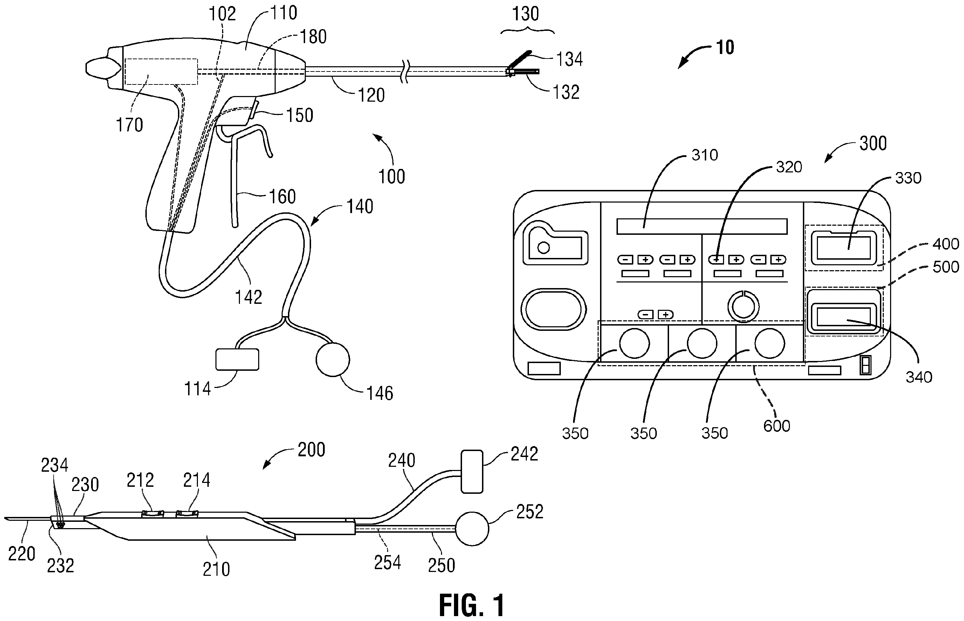

Referring to FIG. 1, a surgical system provided in accordance with aspects of the present disclosure is shown generally identified by reference numeral 10 including a first surgical instrument 100, a second surgical instrument 200, and a surgical generator 300. First surgical instrument 100 is shown and described herein as an ultrasonic surgical instrument incorporating a blade cooling system and second surgical instrument 200 is shown and described herein as an electrosurgical pencil incorporating a smoke evacuation system. However, surgical instruments 100, 200 are merely exemplary and may be configure in any suitable manner; further, additional or alternative surgical instruments, consoles, assemblies, and/or systems may be utilized with surgical generator 300 as part of surgical system 10.

Surgical instrument 100 includes a handle assembly 110, a shaft assembly 120 extending distally from handle assembly 110, an end effector assembly 130 supported at a distal end portion of shaft assembly 120, and a plug assembly 140 operably coupled with handle assembly 110 and extending therefrom for connection to surgical generator 300.

Surgical instrument 100 further includes an activation button 150 mounted on handle assembly 110, a clamp trigger 160 operably coupled to handle assembly 110, and an ultrasonic transducer 170 supported within handle assembly 110. Shaft assembly 120 includes a support sleeve that support a jaw 134 of end effector assembly 130 at a distal end thereof, and a drive sleeve that operably couples to jaw 134. A drive assembly (not shown) couples a proximal portion of the drive sleeve to clamp trigger 160 such that clamp trigger 160 is selectively actuatable to thereby move the drive sleeve to pivot jaw 134 relative to blade 132 of end effector assembly 130 from a spaced-apart position to an approximated position for clamping tissue between jaw 134 and blade 132.

A waveguide 180 is coupled to ultrasonic transducer 170 at a proximal end portion thereof and extends distally through shaft assembly 120 to define blade 132 of end effector assembly 130. Ultrasonic motion produced by ultrasonic transducer 170 is transmitted along waveguide 180 to blade 132 for treating tissue clamped between blade 132 and jaw 134 or positioned adjacent to blade 132.

Surgical instrument 100 further includes a blade cooling system including one or more fluid lines 102 coupled to one or more lumens (not explicitly shown) extending through waveguide 180 and/or blade 132 to circulate cooling fluid therethrough to cool blade 132. The blade cooling system may further include a local, e.g., within or on handle assembly 110, or remote fluid reservoir (not shown). As an alternative to blade cooling, fluid line(s) 102 may be utilized for aspiration and/or irrigation in a configuration where surgical instrument is an ultrasonic aspirator/irrigator.

Plug assembly 140 of surgical instrument 100 includes a cable 142, an ultrasonic plug 144, and a pump plug 146. Ultrasonic plug 144 is configured for connection with ultrasonic plug port 330 of surgical generator 300 while pump plug 146 is configured for connection with one of the pump plug ports 350 of surgical generator 300. Alternatively, plug assembly 140 may include a common plug (not shown) configured to act as both the ultrasonic plug 144 and the pump plug 146. Electrical lead wires electrically coupled to ultrasonic plug 144 extend through cable 142 and into handle assembly 110 for electrical connection to ultrasonic transducer 170 and/or activation button 150 to enable the selective supply of ultrasonic drive signals from surgical generator 300 to ultrasonic transducer 170 upon activation of activation button 150. One or more of fluid line(s) 102 are fluidly coupled to pump plug 146 and extend through cable 142 into handle assembly 110 to connect to the blade cooling system of surgical instrument 100 to enable the pumping of cooling fluid via the pump assembly 600 of surgical generator 300.

Continuing with reference to FIG. 1, surgical instrument 200 includes a body 210 including one or more controls 212, 214 disposed thereon, an electrode probe 220 extending distally from body 210, a smoke evacuation tube 230 extending distally from body 210 and surrounding electrode probe 220 along a portion of the length thereof, and first and second cables 240, 250, respectively.

Electrode probe 220 may be configured to supply monopolar radiofrequency (RF) energy to tissue, although other suitable energies are also contemplated, e.g., bipolar RF, microwave, thermal, ultrasonic etc. Electrode probe 220 may define a sharpened, e.g., needle-shaped, distal tip, a blunt distal tip, and/or any other suitable configuration, e.g., straight, curved, angled, hook-shaped, etc. One or more of the controls 212, 214 control the activation, mode, and/or intensity of energy delivery to electrode probe 220.

Smoke evacuation tube 230, as noted above, surrounds electrode probe 220 along a portion of the length thereof, and defines an open distal end 232 and/or apertures 234 therethrough to facilitate suctioning of fluid, e.g., smoke, therethrough.

First cable 240 includes an electrosurgical plug 242 and houses electrical lead wires electrically coupled to electrosurgical plug 242 and extending through first cable 240 into body 210 to electrically connect to electrode probe 220 and/or controls 212, 214. Electrosurgical plug 242 is configured to connect to electrosurgical plug port 340 of surgical generator 300 to enable the selective supply of electrosurgical energy from generator 300 to electrode probe 220.

Second cable 250 includes a pump plug 252 and defines a suction tube 254 fluidly coupled to smoke evacuation tube 230. Pump plug 252 is configured for connection with one of the pump plug ports 350 of surgical generator 300 to enable the pump assembly 600 of surgical generator 300 to apply suction through suction tube 254 and smoke evacuation tube 230 to facilitate evacuation of fluid e.g., smoke, from a surgical site. An external collection reservoir (not shown) may be coupled to smoke evacuation tube 230 and/or suction tube 254 to collect smoke and/or other fluid evacuated from the surgical site. Alternatively, first and second cables 240, 250 may be combined and extend to a common plug (not shown) or joined at the common plug configured to act as both the electrosurgical plug 242 and the pump plug 252.

Referring still to FIG. 1, surgical generator 300 includes a display 310, a plurality user interface features 320, e.g., buttons, touch-screens, switches, etc., one or more energy ports, e.g., an ultrasonic plug port 330 and a monopolar electrosurgical plug port 340. Other suitable energy ports, e.g., bipolar plug ports, advanced energy plug ports, microwave plug ports, etc., are also contemplated. Surgical generator 300 further includes a plurality of pump plug ports 350.

Surgical generator 300 further includes a ultrasonic generator module 400 configured to generate an ultrasonic drive signal for output through ultrasonic plug port 330 and a monopolar electrosurgical module 500 configured to generate a monopolar RF signal for output through monopolar electrosurgical plug port 340. Of course, where additional or alternate energies are provided, additional or different modules are likewise provided. Surgical generator 300 additionally includes a pump assembly 600 including a plurality of pumps 654 (e.g., first, second ... and "n" pumps 654), configured to provide pump outputs through the plurality of pump plug ports 350 (e.g., first, second ... and "n" pump plug ports 350). Pump assembly 600 is described in greater detail below. Further, in embodiments, one or more common plug ports configured as both a pump plug port 350 and an energy plug port (e.g., an ultrasonic plug port 330, a monopolar electrosurgical plug port 340, a universal smart plug port configured to receive any suitable energy plug and provide appropriate outputs thereto, or any other suitable energy plug port) may be provided.

With respect to control of surgical generator 300, one or more general controllers (not explicitly shown) may be provided for control of the various modules, components, and features of surgical generator 300. Additionally or alternatively, one or more dedicated controllers (not explicitly shown) may be provided for individually controlling one or more of the various modules, components, and features of surgical generator 300.

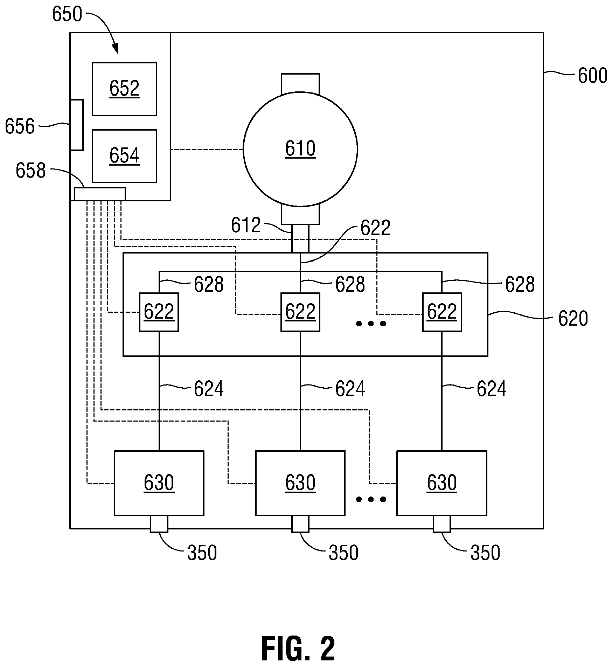

Turning to FIG. 2, pump assembly 600 may be incorporated into surgical generator 300 (FIG. 1) as detailed above, may be incorporated into another surgical component, e.g., instrument, console, system, etc., or may be configured as a stand-alone device. Pump assembly 600 generally includes a motor 610 having a motor output 612, a transmission assembly 620 including a plurality of transmissions 622 (e.g., first, second ... and "n" transmissions 622), each of which is coupled to motor output 612, a plurality of pumps 630 (e.g., first, second ... and "n" pumps 630) coupled to respective transmissions 620, a plurality of pump outputs 640 (e.g., first, second ... and "n" pump outputs 640) coupled between respective pumps 630 and pump plug ports 350, and a controller 650.

Motor 610 may be any suitable motor configured to provide any suitable motor output 612 to transmission assembly 620, e.g., a rotational output, a reciprocating output, rotational and reciprocating outputs, etc. Motor 610, more specifically, is connected to each pump 630 via a separate transmission 622 of transmission assembly 620. Transmission assembly 620 is configured to selectively amplify, attenuate, maintain, and/or convert, if required, the motor output 612 into respective transmission outputs 624 provided to pumps 630. In some instances, transmission assembly 620 may not convert the motor output 612 and, thus, one or more of the transmission outputs 624 may be equal to the motor output 612. Additionally or alternatively, in some instances, transmission assembly 620 may not provide any transmission output 624 from one or more of transmissions 622 regardless of the motor output 612 received.

Transmission assembly 620, as detailed below, enables a single motor, motor 610, to independently and selectively provide different types and/or levels of output to pumps 630 such that each pump 630 may be operated in a desired manner independent of the operation of the other pumps 630a. Further, this configuration also enables the single motor, motor 610, to drive pumps 630 of different types and, further still, enables the motor 610 to provide a single output (rather than a variable output), although variable output motor configurations are also contemplated. One or more of the pumps 630 may be different from the other pumps 630. Alternatively, all of the pumps 630 may be of the same type. The pumps 630, whether the same or different, may include any suitable pump type such as, for example, centrifugal pumps, peristaltic pumps, diaphragm pumps, gear pumps, lobe pumps, roller pumps, piston pumps, screw pumps, vacuum and/or pressure pumps, hydraulic pump, etc.

Continuing with reference to FIG. 2, as noted above, transmission assembly 620 is configured to receive the motor output 612 from motor 610 and selectively provide transmission outputs 624 to pumps 630, which may be the same or of different types and/or levels of output. Motor 610 is described hereinbelow as providing a rotational motor output 612 to transmission assembly 620, although other suitable outputs are also contemplated.

Transmission assembly 620, in embodiments, includes a common input 626 configured to receive the rotational motor output 612 from motor 610. Common input 626 may be coupled to the motor output 612 by, for example, gearing, pulleys, cables, etc., or in any other suitable manner and may be coupled therein to provide a 1:1 input to output ratio, an attenuated output ratio, or an amplified output ratio. Common input 626, in turn, is coupled to a plurality of transmission inputs 628 by, for example, gearing, pulleys, cables, etc., to provide the rotational motor output 612 (whether amplified, attenuated, or maintained) to each of the transmissions 622 via the transmission inputs 628. The couplings between the common input 626 and each of the transmission inputs 628 may provide similar or different output ratios to the transmissions 622, e.g., a 1:1 input to output ratio, an attenuated output ratio, or an amplified output ratio.

Each transmission 622 receives the corresponding transmission input 628 and is selectively connectable to the transmission output 624 for output to drive the corresponding pump 630 in accordance with the transmission output 624 provided thereto. For example, with respect to a rotational output, the rotational speed of the transmission output 624 may be set by the transmission 622, regardless of the motor output 612, to drive the pumps 630 in a desired manner, e.g., at the same or different flow rates, pressures, speeds, etc.

Transmissions 622 may be similar or different and may include any suitable transmission configuration(s) to enable selectively varying the transmission output relative to the transmission input. Suitable transmission configurations include continuously variable transmissions, gearbox transmissions, dual-clutch transmissions, magnetic direct-drive transmissions, and combinations thereof. Regardless of the particular configuration, transmissions 622 may also include disengagement clutches (or other suitable disengagement mechanism) to enable the selective decoupling of the input to the transmission 622 from the output of the transmission 622 to inhibit output regardless of the input.

Each transmission 622 is configured to selectively amplify, attenuate, or maintain, the corresponding transmission input 628 provided thereto for output via the corresponding transmission output 624 to the respective pump 630. Transmissions 622 may additionally be configured to convert the transmission input 628 provided thereto into a different type of output, e.g., converting a rotational input into a reciprocating output or vice versa. Additionally or alternatively, transmissions 622 may be configured to selectively switch the direction of the rotational transmission input 628 compared to the direction of the rotational transmission output 624, e.g., clockwise to counterclockwise or vice versa.

Referring still to FIG. 2, one or more transmissions 622 may be fixed, e.g., providing the same ratio of input to output, and/or one or more transmission 622 may be variable, e.g., enabling continuous or step-wise adjustment of the input to output ratio depending upon the needs of the pump 630 or instrument connected thereto. In this manner, the single motor output 612 from motor 610 may be utilized to drive pumps 630 in similar or different manners and/or at similar or different levels to meet the needs of various different instruments, systems, consoles, etc. connected to pump assembly 600. Deactivation of one or more of the pumps 630 and/or reversal of the direction of one or more of the pumps 630 can also readily be achieved without changing the motor output 612.

Controller 650 includes a processor 652 and memory 654 storing instructions to be executed by the process. Controller 650 may further include an input/output (I/O) 656 and a communication bus 658. I/O 656 enables communication between pump assembly 600 and external modules, components, and/or devices. Communication bus 658 enables communication between, for example, controller 650 and transmissions 622 and pumps 630. In this manner, instructions may be provided, e.g., input into generator 300 and/or received from an instrument or other device connected to one of the pump plug ports 350 (via RFID communication or other suitable detection), based upon a particular detected device connected to one of the pump plug ports 450, to controller 650 via I/O 656 to enable controller 650 to control the configuration of transmission(s) 622, e.g. across communication bus 658, to provide an appropriate output to the corresponding pump(s) 630. Controller 650 may additionally or alternatively receive feedback from pumps 630 via communication bus 658 and, based thereon, control the configuration of transmission(s) 622 to maintain appropriate parameters as part of a feedback-based control loop.

## Claims

1. A surgical generator (300), comprising:
at least one energy module (400, 500) configured to generate an energy-delivery signal;
at least one energy port (330, 340) configured to connect to a surgical instrument to deliver the energy-delivery signal thereto; and
a surgical pump assembly (600) comprising a motor (610) configured to provide a motor output,
**characterized in that**:
the surgical pump assembly further comprises:
a transmission assembly (620) configured to receive the motor output and provide a plurality of transmission outputs, wherein the transmission assembly includes a plurality of transmissions (622) each providing one of the plurality of transmission outputs, and wherein at least one of the transmissions of the plurality of transmissions is controllable to vary the input to output ratio thereof,
a plurality of pumps (630), each transmission of the plurality of transmissions coupled between the motor (610) and a respective one of the pumps of the plurality of pumps (630) whereby each pump of the plurality of pumps is configured to receive a respective one of the transmission outputs of the plurality of transmission outputs; and
a controller (650) disposed in communication with the transmission assembly (620), the controller configured to instruct the transmission assembly to independently vary at least one of the transmission outputs of the plurality of transmission outputs; and
the surgical generator further comprises a plurality of pump output ports (350), each pump output port of the plurality of pump output ports coupled to one of the pumps of the plurality of pumps (630).

2. The surgical generator according to claim 1, wherein at least two of the transmissions (622) of the plurality of transmissions are independently controllable by the controller (650) to independently vary the input to output ratios thereof.

3. The surgical generator according to claim 1 or 2, wherein the at least one energy module comprises an ultrasonic generator module (400) and a monopolar electrosurgical module (500).

## Patentansprüche

1. Chirurgischer Generator (300), umfassend:
mindestens ein Energiemodul (400, 500), das zum Erzeugen eines Energiezufuhrsignals ausgelegt ist;
mindestens einen Energieanschluss (330, 340), der dazu ausgelegt ist, mit einem chirurgischen Instrument verbunden zu werden, um diesem das Energiezufuhrsignal zuzuführen; und
eine chirurgische Pumpenbaugruppe (600), die einen Motor (610) umfasst, der zum Bereitstellen einer Motorleistung ausgelegt ist,
**dadurch gekennzeichnet, dass**:
die chirurgische Pumpenbaugruppe ferner umfasst:
eine Getriebebaugruppe (620), die zum Empfangen der Motorausgabe und Bereitstellen einer Mehrzahl von Getriebeausgaben ausgelegt ist, wobei die Getriebebaugruppe eine Mehrzahl von Getrieben (662) umfasst, die jeweils eine der Mehrzahl von Getriebeausgaben bereitstellen, und wobei mindestens eines der Getriebe der Mehrzahl von Getrieben gesteuert werden kann, um das Eingabe-zu-Ausgabe-Verhältnis davon zu ändern;
eine Mehrzahl von Pumpen (630), wobei jedes Getriebe der Mehrzahl von Getrieben zwischen den Motor (610) und eine jeweilige der Pumpen (630) gekoppelt ist, wobei jede Pumpe der Mehrzahl von Pumpen zum Empfangen einer jeweiligen der Getriebeausgaben der Mehrzahl von Getriebeausgaben ausgelegt ist; und
eine Steuerung (650), die in Kommunikation mit der Übertragungsbaugruppe (620) angeordnet ist, wobei die Steuerung so ausgelegt ist, dass sie die Getriebebaugruppe anweist, mindestens eine der Getriebeausgaben der Mehrzahl von Getriebeausgaben zu ändern; und
der chirurgische Generator ferner eine Mehrzahl von Pumpenausgangsanschlüssen (350) umfasst, jeder Pumpenausgangsanschluss der Mehrzahl von Pumpenausgangsanschlüssen mit einer der Pumpen der Mehrzahl von Pumpen (630) gekoppelt ist.

2. Chirurgischer Generator nach Anspruch 1, wobei mindestens zwei der Getriebe (622) der Mehrzahl von Getrieben von der Steuerung unabhängig (650) gesteuert werden können, um die Eingabe-zu-Ausgabe-Verhältnisse davon zu ändern.

3. Chirurgischer Generator nach Anspruch 1 oder 2, wobei das mindestens eine Energiemodul ein Ultraschallenergiemodul (400) und ein monopolares elektrochirurgisches Modul umfasst.

## Revendications

1. Générateur chirurgical (300), comprenant :
au moins un module d'énergie (400, 500) configuré pour générer un signal de délivrance d'énergie ;
au moins un port d'énergie (330, 340) configuré pour se connecter à un instrument chirurgical pour lui délivrer le signal de délivrance d'énergie ; et
un montage de pompes chirurgicales (600) comprenant un moteur (610) configuré pour fournir une sortie de moteur,
**caractérisé en ce que** :
le montage de pompes chirurgicales comprend en outre :
un montage de transmissions (620) configuré pour recevoir la sortie de moteur et fournir une pluralité de sorties de transmission, le montage de transmissions comportant une pluralité de transmissions (622) fournissant chacune une sortie de la pluralité de sorties de transmission, et au moins une des transmissions de la pluralité de transmissions étant contrôlable pour faire varier le rapport entre l'entrée et la sortie de celle-ci ;
une pluralité de pompes (630), chaque transmission de la pluralité de transmissions étant couplée entre le moteur (610) et l'une respective des pompes de la pluralité de pompes (630), moyennant quoi chaque pompe de la pluralité de pompes est configurée pour recevoir l'une respective des sorties de transmission de la pluralité de sorties de transmission ; et
un contrôleur (650) disposé en communication avec le montage de transmissions (620), le contrôleur étant configuré pour donner l'instruction au montage de transmissions de faire varier indépendamment au moins une des sorties de transmission de la pluralité de sorties de transmission ; et
le générateur chirurgical comprend en outre une pluralité de ports de sortie de pompe (350), chaque port de sortie de pompe de la pluralité de ports de sortie de pompe étant couplé à une des pompes de la pluralité de pompes (630).

2. Générateur chirurgical selon la revendication 1, dans lequel au moins deux des transmissions (622) de la pluralité de transmission sont contrôlables indépendamment par le contrôleur (650) pour faire varier indépendamment les rapports entre l'entrée et la sortie de celles-ci.

3. Générateur chirurgical selon la revendication 1 ou 2, dans lequel l'au moins un module d'énergie comprend un module générateur d'ultrasons (400) et un module électrochirurgical monopolaire (500).
